(19) **Europäisches Patentamt / European Patent Office / Office européen des brevets**

(11) **EP 1 544 618 B1**

(12) **EUROPEAN PATENT SPECIFICATION**

(45) Date of publication and mention
of the grant of the patent:
**18.03.2009 Bulletin 2009/12**

(51) Int Cl.:
*G01N 33/68* (2006.01)    *G01N 33/566* (2006.01)
*G01N 33/53* (2006.01)

(21) Application number: **03771282.5**

(22) Date of filing: **23.07.2003**

(86) International application number:
**PCT/JP2003/009305**

(87) International publication number:
**WO 2004/011948 (05.02.2004 Gazette 2004/06)**

(54) **METHOD OF ANALYZING PROTEIN**

PROTEINANALYSEVERFAHREN

METHODE D'ANALYSE D'UNE PROTEINE

(84) Designated Contracting States:
**AT BE BG CH CY CZ DE DK EE ES FI FR GB GR HU IE IT LI LU MC NL PT RO SE SI SK TR**

(30) Priority: **25.07.2002 JP 2002217099**

(43) Date of publication of application:
**22.06.2005 Bulletin 2005/25**

(73) Proprietor: **Ajinomoto Co., Inc.**
**Tokyo 104-8315 (JP)**

(72) Inventors:
• **Yamada, Naoyuki,**
c/o Ajinomoto Co., Inc.
**Kawasaki-shi, Kanagawa 210-0801 (JP)**
• **Ozawa, Shinichi,**
c/o Ajinomoto Co., Inc.
**Kawasaki-shi, Kanagawa 210-0801 (JP)**
• **Kageyama, Naoko,**
c/o Ajinomoto Co., Inc.
**Kawasaki-shi, Kanagawa 210-0801 (JP)**
• **Miyano, Hiroshi,**
c/o Ajinomoto Co., Inc.
**Kawasaki-shi, Kanagawa 210-0801 (JP)**

(74) Representative: **Nicholls, Kathryn Margaret et al**
**Mewburn Ellis LLP**
**33 Gutter Lane**
**London**
**EC2V 8AS (GB)**

(56) References cited:
WO-A-00/25139              JP-A- 8 338 842
JP-A- 11 503 524          US-A- 5 616 502
US-B1- 6 329 205

• KEMPER COURTENAY ET AL: "An improved, luminescent europium-based stain for detection of electroblotted proteins on nitrocellulose or polyvinylidene difluoride membranes" ELECTROPHORESIS, vol. 22, no. 5, March 2001 (2001-03), pages 881-889, XP002382765 ISSN: 0173-0835
• YAMADA N ET AL: "Detection and quantification of protein residues in food grade amino acids and nucleic acids using a dot-blot fluorescent staining method." JOURNAL OF AGRICULTURAL AND FOOD CHEMISTRY, vol. 52, no. 17, 2004, pages 5329-5333, XP002382766
• LEWIS, S. ET AL.: ECOTOXICOLOGY, vol. 8, October 1999 (1999-10), pages 351-368,
• BRADFORD, M.M.: ANALYTICAL CHEMISTRY, vol. 72, 1976, pages 248-254,
• DATABASE WIKIPEDIA retrieved from HTTP: //EN.WIKIPEDIA.ORG/WIKI Database accession no. http://en.wikipedia.org/wiki/Standard_addition
• BIO-RAD: "Quick Start (TM) Bradford Protein Assay" INSTRUCTION MANUAL,
• ALBA, F.J. ET AL.: ELECTROPHORESIS, vol. 19, no. 14, 1998, pages 2407-2411,

EP 1 544 618 B1

**Description**

TECHNICAL FIELD

The present invention relates to a novel method of analyzing total protein contained in foods, drinks, food additives, medicaments, feeds, etc.

BACKGROUND ART

One of impurities contained in foods and food additives is an allergic substance, and these days this is problematic. For preventing health injury to be caused by such allergen-containing foods, the ordinance relating to the Sanitary Act was revised in April 2001 in Japan, and any one concerned is under an obligation to express the allergic substance in five items of specific raw materials (egg, milk, wheat, buckwheat, and peanut). Since the allergic substance is a protein, the Ministry of Labor, Health and Welfare has shown a guideline that foods having a total protein content of at least (not less than) a few $\mu$g/g require expression of allergic substance therein (see "Interim Report of the Allergy Expression Investigation Society of the Food Expression Study Group" by the Planning Section of the Food Insurance Department of the Drug Bureau of the Ministry of Labor, Health and Welfare, dated October 29, 2001).

Under the background as above, enzyme linked immunosorbent assay (ELISA), is generally employed for detection of allergic substances contained in trace amounts in foods or food additives. In March 2002 just before the enforcement of the ministerial ordinance, Nippon Ham Co. sold ELISA kits for the main 5 items. Similarly, on March 27, 2002, Snow Brand Milk Products Co. announced a method of ELISA for soybean protein in the Agricultural Chemistry Society of Japan (Annual Meeting of the Agricultural Chemistry Society of Japan, 2002). The method comprises extracting a protein from a raw material with a physiological saline solution, immunizing rabbits with it, and establishing an ELISA system by the use of IgG roughly purified through a protein A column. The sensitivity of the method is on a level of 1 ng/ml for soybean and is high, but since the degree of purification of the material and the antibody is low, the selectivity to soybean protein in the method is low. In addition, there is no information relating to the antigen. In the ELISA method, therefore, the antibody to the allergen (antigen) must be first prepared, and a lot of time is taken for the establishment of the analytical method, and, in addition, there is no guarantee that all allergens could be detected. Further, there is still another problem in that the sensitivity and the specificity fluctuate depending on the properties of the antibody used. Moreover, since an antigen-antibody reaction is used in the ELISA method, a neutral solution that enables antigen-antibody bonding must be employed as the sample solution. However, some solid samples could not always have a high solubility at around a neutral pH. For example, some amino acids are hard to be dissolved at around a neutral pH. Tyrosine has an extremely low solubility at around a neutral pH, and therefore the protein detection sensitivity per weight could be from tens to hundreds of ppm even in ELISA.

On the other hand, there is known a method comprising a combination of a dot blot method and a staining method. For example, inadotblotting, ingeneral, the protein concentrated and held on a membrane is visualized by staining with Coomassie brilliant blue or the like as in Bradford M.M. Analytical Biochemistry 72, 248-254 (1976).As another staining method having a high-sensitivity, known is a gold-colloid staining method, which, however, has many drawbacks in that the background is high, staining unevenness may occur, and the operation is complicated. Alba F.J. et al. Electrophoresis 1998, vol. 19, no. 14, pp. 2407-2411 discloses a slot blot method for the analysis of total protein involving fluorescent staining.

Given the situation as above, a method is desired capable of analyzing a protein in trace amounts in a simplified manner with high reproducibility and at high sensitivity.

DISCLOSURE OF THE INVENTION

1. Problems to be solved by the Invention:

The problem to be solved by the present invention is to provide an analytical method for proteins such as allergic substances contained in trace amounts in foods, drinks, food additives, medicaments, feeds, etc, which is a simple, and has a high reproducibility and a high sensitivity.

2. Means for Solving the Problems:

The present inventors have assiduously studied so as to solve the above-mentioned problem, and, as a result, have found that, when a dot blotting method and a fluorescent staining method heretofore employed in the field of biochemistry are combined, then proteins contained in trace amounts in objects to be examined (samples) can be analyzed in a simplified manner at high sensitivity, and, on the basis of this finding, we have completed the present invention.

That is, the present invention, as one embodiment thereof, resides in a method of analyzing total protein in a sample, which comprises subjecting a sample to dot blotting and subsequently to fluorescent staining to stain a protein. The method involves Standard addition.

The analytical method of the present invention enables quantitative analysis and limit analysis (limit test) of the total protein contained in a sample.

The method may include a step of subjecting the sample in a solution state to the dot blotting to thereby fix the protein on a membrane which is used for protein fixation in the sample, and a step of staining the protein fixed on the membrane by the fluorescent staining.

As the sample, employable is any of foods, drinks, food additives, medicaments, feeds and their intermediate products. One preferred example is an amino acid. The method has a sufficient sensitivity for detection of proteins contained in trace amounts in such products.

According to the method, at least 0.1 ppm of protein can be analyzed in a preferred case.

Preferred examples of the membrane for protein fixation are a nitrocellulose membrane and a PVDF (polyvinylidene difluoride) membrane.

As another embodiment thereof, the present invention includes products in which the protein has been analyzed according to the method of the present invention mentioned above, for example, foods, drinks, food additives, medicaments, feeds and their intermediate products, as well as amino acids, etc. Naturally, further, the present invention encompasses the products thus analyzed in the manner as above, or those using them (products).

BRIEF DESCRIPTION OF THE DRAWINGS

[Fig.1]

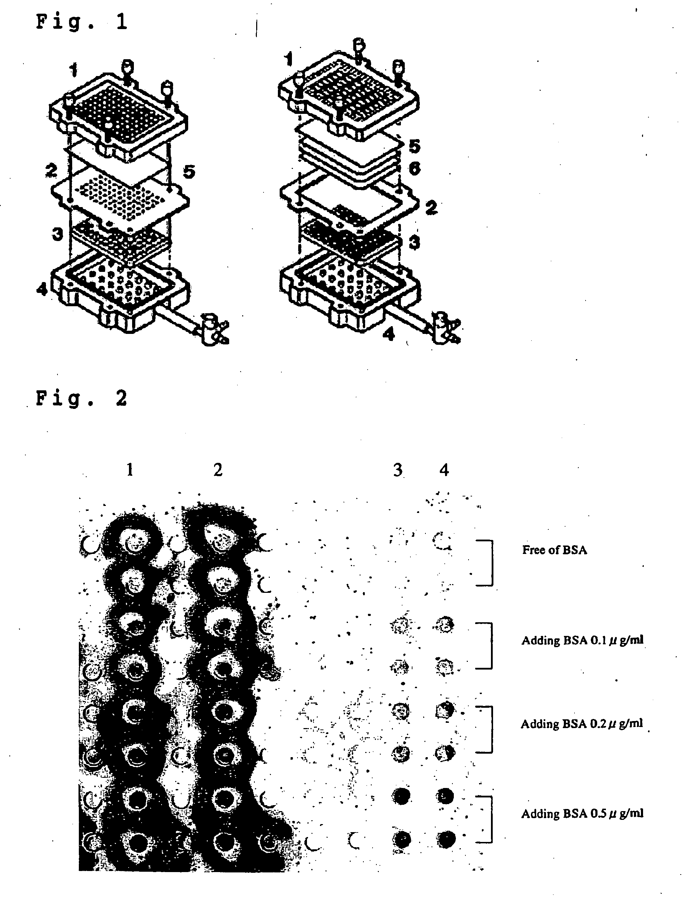

Fig. 1 is an outline view of the dot blotting apparatus used in Example 1.

1. Sample template; 2. Gasket; 3. Gasket support plate; 4. Vacuum manifold; 5. Membrane; and 6. Filter paper.

[Fig. 2]

Fig. 2 shows one example of image data of BSA (bovine serum albumin) preparation and amino acid + BSA sample in Example 1.

Lanes 1, 2: amino acid + BSA sample; Lanes 3, 4: BSA preparation.

[Fig. 3]

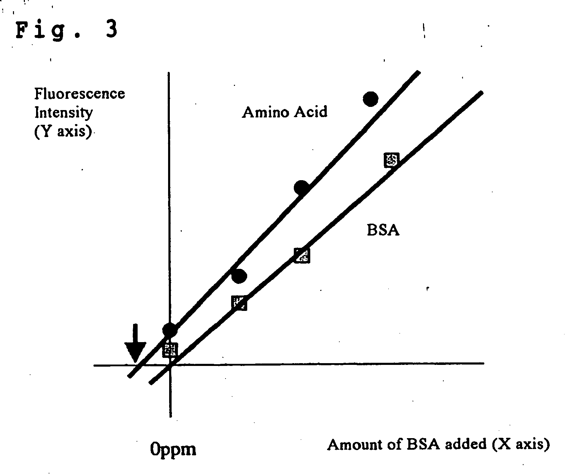

Fig. 3 shows a calculation method of concentration of a protein in amino acid according to a standard addition method in Example 1.

● : Amino acid; ■: BSA; Arrow: Protein concentration in amino acid solution; Horizontal axis: Amount of BSA added; Vertical axis: Fluorescence intensity.

[Fig. 4]

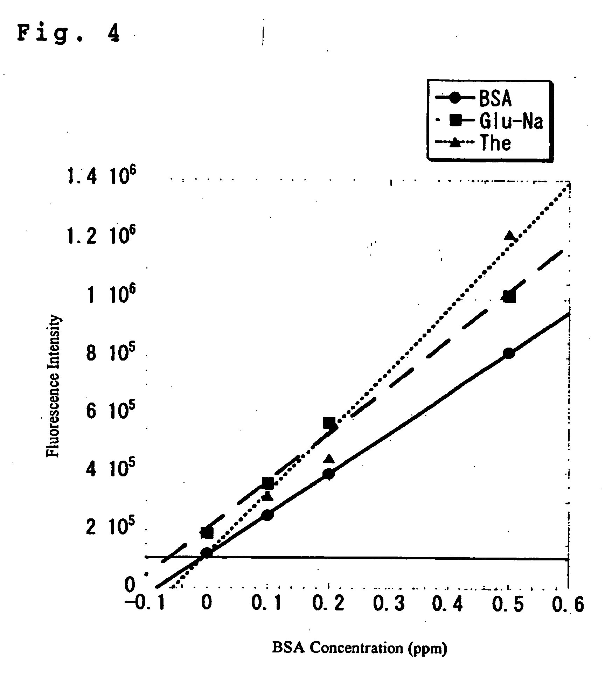

Fig. 4 shows a measurement result of protein (standard addition method) in "Glu-Na" (sodium L-glutamate monohydrate) and "The" (L-theanine), according to a dot blotting-fluorescent staining method in Example 1.
●: BSA; ■: Glu-Na; ▲: The.

Horizontal axis: BSA concentration (ppm); Vertical axis: Fluorescence intensity.

[Fig. 5]

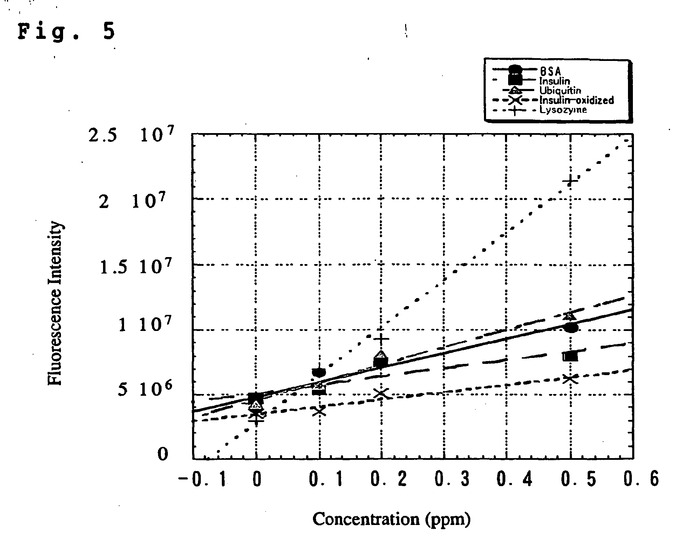

Fig. 5 shows a measurement result of protein in BSA, lysozyme, ubiquitin, insulin and oxidized insulin B-chain, according to a dot blotting-fluorescent staining in Example 2.

● : BSA; ■: Insulin; ▲: ubiquitin; x insulin-oxidized B; +: lysozyme.

Horizontal axis: Protein concentration (ppm); Vertical axis: Fluorescence intensity.

## MODE FOR CARRYING OUT THE INVENTION

The mode for carrying out the invention is described below.

In the present invention, the sample to be analyzed is applied onto a membrane for protein fixation to thereby fix the protein in the sample on the membrane, according to a dot blotting method. In this case, in general, a solution of the sample is subjected to fixation.

One typical example of the dot blotting method comprises putting, for example, a nitrocellulose membrane, a polyvinylidene difluoride membrane (PVDF membrane) or the like between acrylic plates having a large number of holes formed therein, then injecting a protein solution into a hole on the membrane, and subjecting it to suction filtration by aspirator to thereby fix the protein on the membrane. According to the method, it is possible to fix and hold a large number of different types of protein samples on a membrane all at a time while concentrating and desalting them (see Protein Structure Analysis for Gene Cloning, Blotting and Sequencing, written by Hisashi Hirano, published by Tokyo Kagaku Dojin, p. 62).

Next, the protein fixed on the above membrane is stained according to a fluorescent staining, and subsequently this is determined.

A fluorescent staining reagent, SyproRuby recently developed for proteome analysis is a fluorescent staining reagent for protein gel separated through SDS electrophoresis. The advantage of this reagent is that it enables rapid and simple staining at high sensitivity for accurate quantification as compared with conventional methods, or the like. This may be used, for example, for detection or quantification of protein fixed on a PVDF membrane. In protein quantification at a higher sensitivity, the loss may be greater owing to adsorption to vessels or the like in ordinary operation, and detection may be difficult. Accordingly, we have further investigated the way of handling protein contained in trace amounts, and, as a result, have established a high-sensitivity method of quantifying and analyzing protein in which the dilution operation is reduced as much as possible and siliconized containers and chips are used and which enables detection of 0.1 ppm-level protein.

According to the method of the present invention, the combination of dot blotting and fluorescent staining provides a method of analyzing protein contained in trace amounts within a relatively short period of time (approximately 4 or 5 hours from the sample preparation and the image analysis), at high sensitivity for accurate quantification. When the method of the present invention is compared with an ELISA method, then the former may be somewhat inferior to the latter in point of the sensitivity but is superior to the latter in that all the proteins held on the membrane can be detected and determined in the former. Further, smaller proteins could be detected so far as they are highly hydrophobic, and when preferred membranes for protein fixation are selected or developed, then they could also be detected. Regarding solid samples, their detection sensitivity per weight is higher when their solubility is higher. In the present invention, since the pH of the solvent is not specifically defined, any of acid, neutral or alkaline solvent may be used. Therefore, samples may be dissolved in a solvent having a pH value that is the most suitable for the solubility of the samples, and, as a result, the detection sensitivity can be improved.

Quantification and specificity in ELISA method significantly depends on the properties of the antibody used, and also in ELISA method, it is extremely difficult to completely determine all proteins or peptides that may be antigens with no detection failure. In addition, antigens could not be detected when the antibodies to them are not prepared. As opposed to this, the dot blotting-fluorescent staining method according to the present invention enables detection of all i.e. total proteins, and therefore enables evaluation of the proteins irrespective of the presence or absence of antigenicity in protein. Accordingly, it can be said that the method according to the present invention is a measurement method of higher reliability.

Next, the process of the present invention is described briefly. Typical and preferred examples are essentially described hereinunder, but these are typical examples, and the description of these typical examples should not restrict the present invention.

A solution sample is used directly as it is. However, a solid sample is dissolved in a solution having a high solubility to give a possibly highest concentration thereof. Amino acids have a high solubility in acid solutions. Therefore, it is desirable that amino acids are dissolved in aqueous 3.5 % hydrochloric acid solution. For accurately determining the amount of protein in samples according to a standard addition method, samples with a standard protein (e.g., bovine serum albumin, BSA) suitably added thereto are prepared. Next, according to a dot blotting, for example, the protein in the sample is trapped on a nitrocellulose membrane or a PVDF membrane and, at the same time, measurement-disturbing substances are removed from the sample. A 96-well dot blotting apparatus can be purchased from a market, and when this is used, multiple samples can be processed at a time.

After the sample is injected, this is sucked with an aspirator, whereby the protein in the sample is adsorbed by the membrane for protein fixation. Regarding the material of the membrane, a PVDF membrane of high absorbability is preferable. After the liquid has been completely aspirated away, water is injected a few times into it, and then aspirated, whereby the analysis-disturbing substances remaining on the membrane can be removed. Regarding amino acid samples, amino acids reacting with the staining solution, as well as disturbing low-molecular substances and hydrochloric acid and the like can be removed. Next, the washed membrane is taken out of the blotting apparatus (dot blotting apparatus, see Fig. 1), and dried enough.

The membrane thus dried in the manner as above is stained with a fluorescent staining reagent. A fluorescent staining reagent, SyproRuby (manufactured by Molecular Dynamics) developed for proteome analysis is a reagent of high sensitivity and good quantification, and this reagent is preferably used.

The thus-obtained dry membrane is subjected to treatment with acetic acid-methanol solution, whereby the protein is fixed on it, and then the protein is stained with a fluorescent staining reagent. Next, the membrane is washed, and then analyzed preferably by the use of a fluorescent image analyzer.

With the thus-obtained image data, the each sample analyzed and the BSA-added sample thereof are observed as spots. It is recommendable to obtain the area intensity of each spot, and subsequently, plot the data on a graph with the amount of the standard protein on the horizontal axis and the fluorescent area intensity on the vertical axis. In the Examples mentioned below, since the Y-axis section of the blank sample is the background, the test sample data are extrapolated to the line horizontally drawn from it and the section thereof indicates the protein concentration in the test sample. When BSA was used as the standard protein, at least 0.1 ppm of BSA was able to detect: To solution samples, the detection sensitivity can directly apply as it is. On the other hand, the detection sensitivity to solid samples depends on the solubility of the samples. The sample to be analyzed is dissolved in a solvent in which the solubility of the sample is the highest, so that the highest detection sensitivity to the sample can be obtained.

In the method of the present invention, acids and alkalis may be removed after proteins have been adsorbed by membranes. Therefore, the method is also excellent in that it does not receive any limitation on the type of solvents.

Regarding amino acids, even tyrosine and cystine and the like that are neutral and have a low solubility can have an increased solubility when they are acidified.

Recently, in America (US) and also in Japan, foods and food additives that comprise genetically-modified organisms and plants have come on the market, but there still exists deep-seated consumers' anxiety about GMO (genetically-modified organisms). In future, it will be more and more necessary to quantity or detect the protein remaining in food additives produced from GMO, at a sensitivity as high as possible, and to thereby guarantee the quality of products.

Further, the field of regeneration medical treatment that has become much highlighted recently is the most advanced medical technology for regenerating human tissues and organs *in vitro* and transplanting them, and further development of this field in future is more and more expected. In the mediums that are used for regenerating such tissues or organs, since the protein of different species and other persons could be an allergen, protein-free mediums are generally used. Accordingly, also in the field of regeneration medical treatment, methods and means for analyzing or quantifying protein in mediums at a sensitivity as high as possible are desired. In addition to these, it is thought that determinationof protein inmedicaments, cosmetics, supplements (nutrient auxiliary agents, nutrient auxiliary foods) and healthy foods at high-sensitivity will be much more important in future.

For the above-mentioned various requests, the present invention is extremely useful, and is much expected.

<u>PREFERRED EMBODIMENTS</u>

The present invention is described more concretely with reference to the following Examples, to which, however, the present invention should not be limited at all. (Example 1) Quantification of protein in amino acid for food additives:

Devices:

The following devices are used.

Micro-96-well simultaneous filtration apparatus (Atto's AE-6190 Model);
Fluorescent image analyzer (Amersham Biotech's Fluor Imager 595 or Tyhoon 8600);
PVDF membrane (Millipore's PVDF SEQUENCING MEMBRANE, Immobilon-P$^{s0}$);
Shaker (Tokyo Rika's MULTI-SHAKER MMS);
Clean draft (manufactured by Yamato Kagaku);
Aspirator;
Poly-messflask (100 ml);
Siliconized Eppendorf-tube (1.5 ml);
Siliconized chips (250, 1000 μl); and
Micropipetter.

Reagents:

The following reagents are used.

Fluorescent staining solution (Molecular Probe's Ruby protein blot stain, 200 ml bottle);
BSA preparation (Sigma's PROTEIN STANDARD 1000 ppm);
Hydrochloric acid (Kanto Kagaku's ultra-high-purity reagent, 250 ml bottle);
Acetic acid (Junsei Kagaku's special grade reagent, 500 ml bottle); and
Methanol (manufactured by Junsei Kagaku, for high-performance liquid chromatography, 3 liters bottle).

Experiment protocol:

The experiment protocol including the analysis protocol and experiment operation is mentioned below.

Analysis Protocol

1. Washing of devices (washing of wells)
↓
2. Preparation of reagents (3.6 % HCl, fixing solution)
↓
3. Preparation of samples
(Dissolution of amino acid, dilution of BSA preparation, addition of the preparation to amino acid)
↓
4. Cutting and activation of PVDF membrane
↓
5. Dipping in 3.6 % HCl for 30 minutes
↓
6. Setting membrane to blotter, and washing it
↓
7. Addition of sample to membrane
↓
8. Washing of membrane
↓
9. Drying of membrane
↓
10. Fixation of protein on membrane
↓
11. Washing of membrane
↓
12. Staining of membrane
↓
13. Washing of membrane
↓
14. Measurement and analysis with fluorescent imager

Experiment Operation

1. Instruments of sample well, sealing gasket and gasket-holding plate are washed with pure water.
2. Preparation of reagents:

2-1. 3.6 % hydrochloric acid:
10 ml of 36 % hydrochloric acid (Kanto Kagaku's ultra-high-purity reagent, 250 ml bottle) is taken with a micropipetter, and put into a 100-ml volumetric flask of polymeric material, and pure water is added thereto to make 100 ml.
2-2. Fixing solution:
10 ml of methanol (manufactured by Junsei Kagaku, for high-performance liquid chromatography, 3 liters bottle) and 7 ml of acetic acid (Junsei Kagaku's special grade reagent, 500 ml bottle) are taken with a micropipette, and put into a 100-ml poly-messflask, and pure water is added thereto to make 100 ml.

3. Preparation of samples:

3-1. Weigh and dissolution of amino acid:
The following amino acids are dissolved each in 3.6 % hydrochloric acid to have a concentration of 120 mg/ml. (1.5-ml siliconized Eppendorf-tube is used.)
L-Asp, (L-Cys)2, L-Glu, L-Tyr, L-Trp, L-Gln, L-Asp-Na, L-Glu-.Na.
L-Phe is dissolved in 3.6 % hydrochloric acid to have a concentration of 70 mg/ml.
3-2. Preparation of standard reagent (BSA):
BSA is diluted with 3.6 % hydrochloric acid to have a concentration of 0, 0.1, 0.2, or 0.5 $\mu$g/ml. "0 $\mu$g/ml" is 3.6 % hydrochloric acid.
3-3. Preparation of amino acid solution (SA) of test sample:
Test samples are diluted each with an amino acid solution so that the BSA concentration in SA could be 0, 0.1, 0.2 or 0.5 $\mu$g/ml. "0 $\mu$g/ml" is amino acid solution.

4. Cutting and activation of PVDF membrane:
A PVDF membrane is cut with scissors into a piece of approximately 12 cm x 9 cm, and its front and back faces are marked top right with a pencil so as to be differentiated from each other. Methanol is put into a vessel, and the membrane piece is dipped therein for 1 minute.
5. Dipping in 3.6 % HCl for 30 minutes:
Hydrochloric acid is put into a vessel, and the activated PVDF membrane is dipped in 3.6 % hydrochloric acid for 30 minutes.
6. Setting membrane to blotter, and washing it:
The membrane is set in a blotter, and 350 $\mu$l of 3.6 % hydrochloric acid is applied thereto. Under suction with an aspirator, this is washed. An outline of the blotting apparatus (dot blotting apparatus) used herein is shown in Fig. 1.
7. Applying of sample to membrane:
350 $\mu$l of SA is applied to the membrane, and sucked with an aspirator.
8. Washing of membrane:
350 $\mu$l of 3 . 6 % hydrochloric acid is applied to the membrane, and the membrane is washed with it under suction with an aspirator. Next, 350 $\mu$l of pure water is applied to the membrane thus washed, and the membrane is washed with it under suction with an aspirator.
9. Drying of membrane:
The membrane is released from the blotter (blotting apparatus), and dried in clean draft for 1 hour.
10. Fixation of protein on membrane:
The fixing solution is put into a vessel for its exclusive use, and the dry membrane is floated on the fixing solution with its transfer surface facing down. Using a shaker, this is fixed for 15 minutes. (The speed of the shaker is 50 rpm.)
11. Washing of membrane:
Pure water is put into a vessel. The membrane is floated on the pure water with its transfer surface facing down. This is washed for 5 minutes, using a shaker. (The speed of the shaker is 50 rpm.) The membrane is taken out, and pure water in the vessel is exchanged, and the membrane is again washed. The washing operation with pure water is repeated 4 times.
12. Staining of membrane:
A staining solution is put into a vessel. The membrane is floated on the staining solution with its transfer surface facing down. This is stained for 15 minutes, using a shaker. (The speed of the shaker is 50 rpm.)
13. Washing of membrane:
After 15 minutes, the membrane is taken out, and pure water is put into the vessel. The membrane is floated on the pure water with its transfer surface facing down. This is washed for 1 minute, using a shaker. (The speed of the shaker is 50 rpm.) The membrane is taken out, and pure water in the vessel is exchanged, and the membrane is again washed. The washing operation with pure water is repeated 2 times.
14. Measurement and analysis with fluorescent imager:
14-1. Method of using fluorescent imager:
Measurement condition and measurement protocol are described below.
Measurement Condition:

Emission Filter:    Rox610BP30
PMT:                415
Laser:              Green (532 nm)

(continued)

Sensitivity:          Normal

Measurement Protocol:

(a) The cover of the body is opened, and the PVDF membrane is put on the glass plate. In this stage, the stained surface of the membrane is made to face down (on the glass side).
(b) A non-fluorescent glass plate is put on the membrane to press it.
(c) The body cover is closed.
(d) The scanning range is defined, and the measurement is started.
(e) After the scanning is finished, the data are stored.
(f) In case of sensitivity insufficiency or fluorescent saturation, after changing the PMT voltage value, the measurement is again carried out.

14-2. Imager:
Using a software purchased on a market, the image data are analyzed. As in Fig. 2, the spots are circled and the integral value of the fluorescent intensity in the circles is obtained.

(Result)

One example of the image obtained according to the dot blotting method is shown in Fig. 2. As in Fig. 2, the spot concentration of the BSA preparation increases with the increase in the amount thereof. A spot in added amount 0.1 $\mu$g/ml (0.1 ppm) of BSA is obviously observed. Therefore, this confirms that the present invention enables detection of at least 0.1 ppm protein. The same spot concentration change is also observed in the actual amino acid samples, and in addition, doughnut-like fluorescent circles concentric with sample spots are observed. This may be because the amino acid wetted around the wells while removed under reduced pressure would have reacted with the fluorescent reagent. Accordingly, in quantitative analysis, it has been decided that the product (volume) of the fluorescent intensity in the spot part is adopted. As a result, in the addition and recovery experiment of BSA and the each amino acids, linearity is seen in them though the inclination differs. The reasons why the inclination differs depending on the type of the amino acid will be two, <1> there is a difference in the adsorbed amount of BSA to the instruments or the like, owing to the presence of the amino acid, and <2> the amino acid is adsorbed by BSA to remain. Accordingly, based on the BSA line and the line running through the Y-axis of the blank (without BSA addition), which are measured on the same membrane, the intersections with the line of each amino acid according to the standard addition method are defined as the protein concentration (see Fig. 3). The protein concentration in the actual samples is calculated according to the following formula, using on the dilution ratio of each amino acid.

```
Protein concentration in actual sample

= (protein concentration in solution obtained according to

standard addition method) × (dilution ratio).
```

Dilution ratio: 14.3 times for Asp, Cys, Glu, Phe, Tyr, Gln, Trp; and 8.3 times for the others than these.
As a result, at least 0.1 ppm of the bovine serum albumin (BSA) used as a preparation, and at least 1 ppm of the amino acid in terms of the concentration thereof in each sample can be detected.
Fig. 3 shows a method of calculating the protein concentration in amino acid according to a standard addition method, in which the arrow indicates the protein concentration in an amino acid solution.
According to this method, 25 items of amino acids for food additives were analyzed. A part of the results are shown in Fig. 4; and a part of the quantification results of those 25 items are shown in Table 1. In the samples of all those 25 items of amino acids, the protein concentration was not more than 1 ppm (not more than 1 $\mu$g/g).
Fig. 4 shows measurement results (standard addition method) of protein in amino acids for food additives, GluNaH$_2$O (sodium L-glutamate monohydrate) and The (L-theanine), according to a dot blotting-fluorescent staining method.

[Table 1] Quantification Results of Protein in Amino Acids for Food Additives

| Abbreviations | Designation | Analysis Result according to Dot Blotting-Fluorescent Staining method |
|---|---|---|
| L-Ala | L-alanine | not more than 1 ppm |
| (L-Cys)$_2$ | L-cystine | not more than 1 ppm |
| L-GluNaH$_2$O | sodium L-glutamate monohydrate | not more than 1 ppm |
| L-Ile | L-isoleucine | not more than 1 ppm |
| L-Leu | L-leucine | not more than 1 ppm |
| L-Thr | L-threonine | not more than 1 ppm |
| L-Tyr | L-tyrosine | not more than 1 ppm |
| L-Val | L-valine | not more than 1 ppm |
| L-CysHClH$_2$O | L-cysteine hydrochloride monohydrate | not more than 1 ppm |
| L-Gln | L-glutamine | not more than 1 ppm |
| L-Trp | L-tryptophane | not more than 1 ppm |
| L-Arg | L-arginine | not more than 1 ppm |
| DL-Ala | DL-alanine | not more than 1 ppm |
| L-The | L-theanine | not more than 1 ppm |

The above-mentioned results confirm that, according to the method, protein in amino acid can be analyzed at high sensitivity. In particular, it is understood that the dot blotting is effective which uses a PVDF membrane as a membrane for protein fixation thereon and uses a fluorescent staining reagent. As a result of analysis of 25 items of amino acids for food additives with this method, it has been clarified that the protein concentration in all these items of samples is not more than 1 ppm.

In this Example, the amino acid concentration is kept constant. However, in case of amino acids having high solubility, by making the amino acid concentration high, the detection sensitivity can be further increased.

(Example 2) Molecular weight range of detectable protein:

Protein having a large molecular weight becomes allergen easily, and such high-molecular-weight protein is readily detected in ELISA. On the other hand, even protein having a lower molecular weight may be allergen, so that it is desirable that protein having a lower molecular weight and peptide could be detected at high sensitivity. According to the method of the present invention, proteins having a different molecular weight, BSA (66 kDa), lysozyme (14 kDa), ubiquitin (8.6 kDa), insulin (5.7 kDa) and oxidized insulin B chain (3.5 kDa) were tested for the minimum limit of detection and the quantification ability thereof. In place of BSA in Example 1, each protein was suitably diluted and its calibration curve was formed according to the dot blotting-fluorescent staining method. As a result (see Fig. 5), all proteins had calibration curves of high linearity, and their minimum limit of detection was 0.1 ppm (but the minimum limit of detection of oxidized insulin B chain was only 0.2 ppm). This confirmed a detection of even proteins having a low molecular weight at high-sensitivity.

EFFECT OF THE INVENTION

As described hereinabove, the present invention provides a method of analyzing total protein. According to the method, a protein such as allergic substance contained in trace amounts in foods, drinks, food additives, medicaments, cosmetics, feeds, etc. can be analyzed in a simplified manner at high sensitivity. Accordingly, the present invention can be carried out widely in various industrial fields especially in those of foods, medicaments, cosmetics, feeds, etc., and its usefulness is obvious.

## Claims

1. A method of quantitative analysis of total protein in a solution according to a standard addition method in which a standard protein is added to samples of the solution, and the samples are then subjected to dot blotting on a membrane, and subsequently to staining with a fluorescent staining reagent, and to determination of the staining.

2. A method according to claim 1 wherein the standard protein added is bovine serum albumin.

3. A method according to claim 2 wherein bovine serum albumin addition to the sample is at a level of 0 to 0.5 $\mu$g/ml.

4. A method according to any one of the preceding claims wherein the membrane is a nitrocellulose membrane or a PVDF membrane.

5. The method of any one of claims 1 to 4 wherein the solution is a food, drink, food additive, medicament or feed, or wherein the solution is produced by dissolving a solid sample of a food, drink, food additive, medicament or feed.

6. A method according to any one of the preceding claims wherein the solution analysed is produced by dissolving an amino acid sample.

7. A method according to claim 6 wherein the amino acid sample for dot blotting is dissolved in an acid solution.


## Patentansprüche

1. Verfahren der quantitativen Analyse von Gesamtprotein in einer Lösung nach einem Standard-Additionsverfahren, bei dem ein Standard-Protein zu Proben der Lösung hinzugefügt wird und die Proben anschließend einem Dot-Blotting auf einer Membran sowie anschließendem Färben mit einem fluoreszierenden Färbereagens sowie einer Bestimmung der Färbung unterzogen werden.

2. Verfahren nach Anspruch 1, worin das hinzugefügte Standard-Protein Rinderserum-Albumin ist.

3. Verfahren nach Anspruch 2, worin die Rinderserum-Albumin-Zugabe zu der Probe in einem Ausmaß von 0 bis 0,5 $\mu$g/ml erfolgt.

4. Verfahren nach einem der vorangegangenen Ansprüche, worin die Membran eine Nitrocellulose-Membran oder eine PVDF-Membran ist.

5. Verfahren nach einem der Ansprüche 1 bis 4, worin die Lösung ein Nahrungsmittel, ein Getränk, ein Nahrungsmittel-Additiv, ein Medikament oder ein Futtermittel ist oder worin die Lösung durch Auflösen einer festen Probe eines Nahrungsmittels, Getränks, Nahrungsmittel-Additivs, Medikaments oder Futtermittels produziert wird.

6. Verfahren nach einem der vorangegangenen Ansprüche, worin die analysierte Lösung durch Auflösen einer Aminosäure-Probe produziert wird.

7. Verfahren nach Anspruch 6, worin die Aminosäure-Probe für das Dot-Blotting in einer sauren Lösung aufgelöst wird.


## Revendications

1. Méthode d'analyse quantitative d'une protéine totale dans une solution selon une méthode d'addition standard dans laquelle une protéine standard est ajoutée à des échantillons de la solution, et les échantillons sont ensuite soumis à une hybridation sur tache sur une membrane et ensuite à une coloration avec un réactif de coloration fluorescent et à la détermination de la coloration.

2. Méthode selon la revendication 1, où la protéine standard ajoutée est de l'albumine du sérum bovin.

3. Méthode selon la revendication 2, où l'addition de l'albumine du sérum bovin à l'échantillon se situe à un niveau de 0 à 0,5 $\mu$g/ml.

**4.** Méthode selon l'une quelconque des revendications précédentes, où la membrane est une membrane de nitrocellulose ou une membrane PVDF.

**5.** Méthode selon l'une quelconque des revendications 1 à 4, où la solution est un aliment, une boisson, un additif d'aliment, médicament ou aliment, ou bien où la solution est produite en dissolvant un échantillon solide d'un aliment, boisson, additif d'aliment, médicament ou aliment pour animaux.

**6.** Méthode selon l'une quelconque des revendications précédentes, où la solution analysée est produite en dissolvant un échantillon d'acides aminés.

**7.** Méthode selon la revendication 6, où l'échantillon d'acides aminés pour l'hybridation sur tache est dissout dans une solution acide.

Fig. 1

Fig. 2

| | | | Free of BSA |
| | | | Adding BSA 0.1 μ g/ml |
| | | | Adding BSA 0.2 μ g/ml |
| | | | Adding BSA 0.5 μ g/ml |

# Fig. 3

Fluorescence
Intensity
(Y axis)

Amino Acid

BSA

0ppm

Amount of BSA added (X axis)

Fig. 4

# Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **BRADFORD M.M.** *Analytical Biochemistry,* 1976, vol. 72, 248-254
- **ALBA F.J. et al.** *Electrophoresis,* 1998, vol. 19 (14), 2407-2411
- **HISASHI HIRANO.** Protein Structure Analysis for Gene Cloning. Tokyo Kagaku Dojin, 62